# EUROPEAN PATENT SPECIFICATION

(11) **EP 3 687 631 B1**
(45) Date of publication and mention of the grant of the patent: **10.11.2021**
(21) Application number: 18769967.3
(22) Date of filing: 10.09.2018
(51) Int. Cl.: A61Q 5/06, A61K 8/04, A61K 8/34, A61K 8/37, A61K 8/44, A61K 8/81

(54) **HAIR STYLING COMPOSITION CONTAINING VINYL CAPROLACTAM/VP/DIMETHYLAMINOETHYL METHACRYLATE COPOLYMER**
HAARSTYLINGZUSAMMENSETZUNG MIT VINYL-CAPROLACTAM/VP/DIMETHYLAMINOETHYL-METHACRYLAT-COPOLYMER
COMPOSITION DE COIFFAGE CONTENANT UN COPOLYMÈRE DE VINYLCAPROLACTAME/VP/MÉTHACRYLATE DE DIMÉTHYLAMINOÉTHYLE

(30) Priority: 30.09.2017 WO PCT/CN2017/104754
(43) Date of publication of application: 05.08.2020
(73) Proprietor: Beiersdorf AG, 20253 Hamburg (DE)
(72) Inventor: WU, Shuiping, Wuhan 430056 (CN); LIU, Xiaoping, Wuhan 430060 (CN); CAO, Mi, Wuhan 430073 (CN); WANG, Fan, Wuhan 430065 (CN); BAO, Zhaoxia, Wuhan 430070 (CN)
(74) Representative: Beiersdorf AG
(86) International application number: PCT/EP2018/074245
(87) International publication number: WO 2019/063268

(56) References cited:
- EP-A2- 0 074 191
- US-A- 5 221 531
- US-A1- 2015 104 404

## Description

### Technical field

The invention relates to a cosmetic field, in particular a hair styling composition in form of a hair gel spray (sprayable hair gel), comprising a Vinyl Caprolactam/VP/Dimethylaminoethyl Methacrylate Copolymer. The invention provides a very attractive composition in terms of a clear gel spray and a very pleasant sensory impression upon use. The cosmetic composition leads to a very satisfying hair styling.

### Background Art

The general aim of hair styling is to provide strong hold together with a natural appearance of the styled hair. The hair fixing agents should be easily brushed and/or washed out, leaving the hair in a non-dry, non-damaged and pleasant condition.

Hair styling compositions may have different galenic forms, including sprays, foams, gels, waxes and liquids.

Hair spray compositions are aerosol compositions or pump sprays. Hair sprays are used to fix a hair style and to protect hair from wind, sun, and humidity. In general, hair sprays contain a film-forming/fixing polymer, a solvent, and a propellant, which is air for pump sprays. Additionally, suitable ingredients may be contained, for instance UV filters.

Hair gels' function is to fix hair. Hair gels may be described as thickened hair fixing solutions with varying degrees of fixing. In general, they are water-based and contain a film-forming/fixing polymer, a thickening polymer, and additionally suitable cosmetic ingredients. After application on the hair, the gels dry, leaving a film on the hair. This film will provide the styled hair with a strong and long-lasting hold.

Hair gels may also impart hair with a wet looking appearance, such hair gels are called wet look hair gels.

A composition combining both galenic forms, namely hair spray and hair gel, is a hair gel spray. In general, hair gel sprays contain a film-forming/fixing polymer, a solvent, a small amount of thickener and a propellant. Gel sprays are in form of aerosol sprays and pump sprays.

Prior art mostly discloses hair spray compositions or hair gel compositions. However, a few examples are found describing hair gel spray compositions.

EP 1685827 B1 discloses rheology modifiers in hair gel compositions. One example (example 3) describes a "Spray Hair Gel", comprising PVP as a fixing polymer.

US 2007/0248561 A1 discloses water-soluble polymers and their cosmetic use. Hair styling gels, which are sprayable are described. A number of different non-crosslinked polymers and a number of different fixing polymers, such as an amine oxide methacrylate copolymer (Diaformer® Z 651 N and Diaformer® Z 712 N), PVP polymer and VP/VA polymer, are disclosed. The main issue of EP 2301517 A1 is the disclosure of compositions containing particles comprising a wall material and a core material. However, an example describing a spray hair gel containing PVP as a fixing polymer is also disclosed. Hair styling compositions containing particles are described in WO 03/028676 A1. A spray-on gel hair composition is disclosed comprising polyquaternium-11.

None of the aforementioned documents discloses the use of a Vinyl Caprolactam/VP/Dimethylaminoethyl Methacrylate Copolymer, a very suitable film-forming/fixing polymer in a hair gel spray composition.

WO 2005/092276 A1 describes the use of Vinyl Caprolactam/VP/Dimethylaminoethyl Methacrylate Copolymer. However the respective example only discloses a composition containing Vinyl Caprolactam/VP/Dimethylaminoethyl Methacrylate Copolymer, a polymer AF, water, glycolic acid and a non-characterized preservative. A comparable disclosure is present in WO 2010/02097 A1. The resulting gels are suitable for hair setting and are described as clear gels. However, different and further components have to be added to hair gel sprays, which are intended to be launched in the market. For instance, the addition of some preservatives and/or perfume mixtures resulted in opaque, cloudy compositions. Furthermore, increasing the temperature, especially a temperature above 40°C, resulted in opaque and cloudy compositions, too. These opaque and cloudy compositions are not really accepted by the consumer.

The Vinyl Caprolactam/VP/Dimethylaminoethyl Methacrylate Copolymer is a water-soluble film-former, illustrated by the following chemical structure: 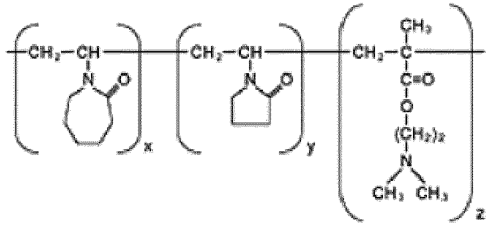 Wherein x, y, and z are each > 1, but preferably > 10.

Aqueous solutions of Vinyl Caprolactam/VP/Dimethylaminoethyl Methacrylate Copolymers are known to have low cloud point temperatures. If the temperatures increases above 40°C, the solutions become cloudy or hazy. Experiments have been described to raise the cloud point temperature. Aqueous solutions of a specific Vinyl Caprolactam/VP/Dimethylaminoethyl Methacrylate Copolymer were combined with various surfactants belonging to different classes, namely anionic, cationic and nonionic surfactants. The addition of specific surfactants, such as sodium lauryl sulfate, sodium lauryl ether sulfate, sarcosinate, cetrimonium chloride, oleth-20 and palmitamidopropyltrimonium chloride, resulted in an elevation of the cloud point temperature of an aqueous solution of Vinyl Caprolactam/VP/Dimethylaminoethyl Methacrylate Copolymer (Technical information Advantage™ S polymer, Ashland, 2013).

However, the cloud point temperature is not only dependent on the Vinyl Caprolactam/VP/Dimethylaminoethyl Methacrylate Copolymer, but also on the combination of Vinyl Caprolactam/VP/Dimethylaminoethyl Methacrylate Copolymer with further ingredients, which have to be added to compositions, intended to be launched in the market.

The problem to be solved is to provide appealing, especially clear, styling compositions, especially hair gel sprays, characterized by an elevated cloud point temperature. Said compositions should help to style hair in the desired way and should provide good and long-lasting hold to the styled hair. Furthermore, even after repeated application, hair should not become dry, thin and/or brittle, instead stay healthy, shiny and easy manageable.

### Summary of the invention

The aforementioned problem is solved by a cosmetic composition, in particular a hair styling composition in form of a hair gel spray, comprising
a. a Vinyl Caprolactam/VP/Dimethylaminoethyl Methacrylate Copolymer,
b. at least one salt of an acyl amino acid, salt of creatine and/or glutamate,
c. at least one preservative, selected from parabens and/or phenoxyethanol,
d. optionally at least one solvent.

Furthermore, the composition can optionally comprise auxiliary components as listed in the part of "detailed description of the invention", such as antioxidants.

According to the invention the composition may contain water as a solvent.

In addition to a Vinyl Caprolactam/VP/Dimethylaminoethyl Methacrylate Copolymer, at least one salt of an acyl amino acid, salt of creatine and/or glutamate, at least one preservative, selected from parabens and/or phenoxyethanol and optionally one or more solvents, e.g., water, ethanol, and PEG-40 hydrogenated castor oil, are contained to bring the total amount of all components to 100% by weight.

In another aspect, the present invention refers to use of at least one salt of an acyl amino acid, salt of creatine and/or glutamate to raise the cloud point temperature of a composition containing a Vinyl Caprolactam/VP/ Dimethylaminoethyl Methacrylate Copolymer and at least one preservative, selected from the group of parabens and phenoxyethanol, resulting in an appealing clear composition at an elevated temperature

Furthermore, the present invention also refers to use of composition of the present invention for styling and caring hair, especially providing styled hair with a long-lasting hold. After use, the hair is not sticky and remains in moisturized and cared condition.

By using the composition of the present invention, the disadvantages of the prior art compositions containing Vinyl Caprolactam/VP/Dimethylaminoethyl Methacrylate Copolymers are overcome. The styling compositions of the present invention lead to significantly improved compositions referring to transparency. Furthermore, the hold of the styled hair together with providing moisture and care is improved, too.

### Detailed description of the invention

According to the invention a Vinyl Caprolactam/VP/Dimethylaminoethyl Methacrylate Copolymer is contained in hair styling compositions, in particular hair gel spray compositions. Vinyl Caprolactam/VP/Dimethylaminoethyl Methacrylate Copolymer may be purchased for instance as Copolymer VC-713 (trade name) from Ashland, Inc.

Advantageously, Vinyl Caprolactam/VP/Dimethylaminoethyl Methacrylate Copolymer is contained in the compositions of the present invention in an amount of 0.1 to 8.0% by weight, preferably 0.3 to 4.0 % by weight, based on the total weight of the composition.

Acyl amino acids are derived from amino acids. The acyl group may be attached to the amino group or a further group of the amino acid. There are several amino acids, wherein glutamate and sarcosinate are preferably selected.

The respectively preferably selected acyl amino acids are acyl glutamate and acyl sarcosinate, e.g., its sodium or potassium salt.

More preferably acyl glutamate may be contained in form of lauroyl glutamate or cocoyl glutamate in the compositions of the present invention. In an embodiment, acyl glutamate is sodium acyl glutamate, e.g., sodium lauroyl glutamate or sodium cocoyl glutamate. Sodium lauroyl glutamate may be purchased as ULS-30S from Guangzhou Naco Chemical Co., Ltd and sodium cocoyl glutamate may be purchased as AMIN GC95F from Guangzhou Tinci Materials Technology Co., Ltd.

As well, more preferably acyl sarcosinate may be contained in form of lauroyl sarcosinate in the compositions of the present invention. In an embodiment, acyl sarcosinate is sodium acyl sarcosinate, e.g., sodium lauroyl sarcosinate. Sodium lauroyl sarcosinate may be purchased as AMIN LS30-NP from Guangzhou Tinci Materials Technology Co., Ltd.

The amino acid derivative creatine in form of its salt, e.g., sodium or potassium salt, may also preferably be contained in the compositions according to the present invention. The sodium salt of creatine may be purchased from Guangzhou Tinci Materials Technology Co., Ltd.

The amino acid glutamate in form of its salt, e.g., sodium or potassium salt, may also preferably be contained in the compositions according to the present invention. Sodium glutamate may be purchased from Guangzhou Tinci Materials Technology Co., Ltd.

All the aforementioned components have an influence on the cloud point temperature of the compositions according to the present invention containing a Vinyl Caprolactam/VP/Dimethylaminoethyl Methacrylate Copolymer.

All the aforementioned components are preferred and may be summed up in the group of lauroyl glutamate, cocoyl glutamate, lauroyl sarcosinate, salt of creatine, and glutamate, specifically, sodium lauroyl glutamate, sodium cocoyl glutamate, sodium lauroyl sarcosinate, sodium salt of creatine, and sodium glutamate. Each of the said components may be contained alone in the compositions of the present invention or mixtures of the components may be contained.

The at least one salt of an acyl amino acid, salt of creatine, and/or glutamate may be contained in an amount of 0.1 to 3.0 % by weight, preferably 0.4 to 2.0 % by weight, relative to the total weight of the composition. The amounts given are referring to the total weight of one of the substances of the group of lauroyl glutamate, cocoyl glutamate, lauroyl sarcosinate, salt of creatine, and glutamate or to the total weight of mixtures of the substances of the group of lauroyl glutamate, cocoyl glutamate, lauroyl sarcosinate, salt of creatine, and glutamate, specifically, sodium lauroyl glutamate, sodium cocoyl glutamate, sodium lauroyl sarcosinate, sodium salt of creatine, and sodium glutamate in the compositions according to the invention.

The hair styling compositions of the present invention comprise at least one preservative, selected from the group of preservatives, which are allowed and suitable for cosmetic preparations. At least one paraben and/or phenoxyethanol is used.

Parabens may be characterized by the following structure:

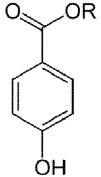

Wherein R is a substituent representing alkyl substituents with 1 to 5 carbon atoms or an aromatic substituent. The alkyl substituent may be straight or branched. Suitable parabens are methyl paraben, ethyl paraben, propyl paraben, butyl paraben, as well as isopropyl paraben, isobutyl paraben, pentyl paraben and phenyl paraben. The compositions according to the invention may contain one paraben or a mixture of parabens. Preferably, methyl paraben and/or ethyl paraben are contained in the compositions according to the present invention.

In a further preferred embodiment the compositions according to the present invention contain phenoxyethanol.

In a further preferred embodiment the compositions according to the present contain phenoxyethanol and one or more parabens, preferably methylparaben and/or ethyl paraben.

The at least one paraben may be contained in an amount of 0.01 to 0.5 % by weight, preferably 0.05 to 0.25 % by weight, relative to the total weight of the composition. The amounts given are referring to the total weight of one paraben or more parabens in the compositions according to the invention.

Phenoxyethanol may be contained in an amount of 0.1 to 1.5 % by weight, preferably 0.2 to 1.0 % by weight, relative to the total weight of the composition.

Solvents may be necessary to dissolve ingredients in order to facilitate the incorporation of these ingredients in the final composition. If, for instance, the compositions are water-based, lipophilic ingredients may be difficult to incorporate. Solvents help to incorporate these ingredients.

According to the invention the following solvents may be contained:
- water;
- oils such as triglycerides of capric or caprylic acid, preferably castor oil;
- ethoxylated triglycerides which may be hydrogenated, such as PEG-40 hydrogenated castor oil;
- fats, waxes and other natural and synthetic lipids, preferably esters of fatty acids with alcohols of low C number (e.g., C1-6), for example with isopropanol, propylene glycol or glycerol, or esters of fatty alcohols with alkanolic acids of low C number (e.g., C1-6) or with fatty acids;
- alcohols, diols or polyols of low C number (e.g., C1-6) and their ethers, preferably ethanol, isopropanol, propylene glycol, glycerol, ethylene glycol, ethylene glycol monoethyl ether or ethylene glycol monobutyl ether, propylene glycol monomethyl ether, propylene glycol monoethyl ether or propylene glycol monobutyl ether, diethylene glycol monomethyl ether or diethylene glycol monoethyl ether, and analogous products.

Mixtures of the abovementioned solvents may be used.

According to the invention the compositions preferably contain water, ethanol and/or PEG 40 hydrogenated castor oil.

Advantageously, the solvents selected from ethanol and/or PEG 40 hydrogenated castor oil are present in amounts of 0.1 to 7.5 % by weight, preferably 0.2 to 5.5 % by weight, relative to the total weight of the composition. The amounts given are referring to the total weight of a solvent or mixtures of solvents in the compositions according to the invention.

Alternatively, in further embodiments the amount of ethanol contained in the compositions of the present invention may be higher than 7.5 % by weight, amounts up to 50 % by weight or ethanol may be used as a balance compound, so that all the components of a composition amount to 100% by weight.

According to the present invention at least one antioxidant may be used in the hair styling composition. In general, antioxidants are molecules which prevent other molecules from being oxidized. Oxidation processes may produce free radicals which are damaging or destroying other molecules. When these other molecules are part of living cells, these cells can be damaged. There are several molecules which are able to function as radical scavenger. These molecules are also called antioxidants. The antioxidants can be of natural origin as for example vitamin C, gluthathione, lipoic acid, carotenes, vitamin E and coenzyme Q. But there are also synthetic antioxidants as butylhydroanisol (BHA), butylhydroxytoluol (BHT) and pentaerythrityl tetra-di-t-butyl-hydroxyhydrocinnamate. Benzophenone-4 may also be used as an antioxidant. Benzophenone-4 normally is an UV filter, protecting the skin from damages caused by UVB and short-wave UVA ultraviolet light. According to the invention, Benzophenone-4 is preferred to be used as antioxidant of synthetic origin.

The at least one antioxidant is used in an amount of 0.001 to 0.1 % by weight, preferably 0.01 to 0.07 % by weight, relative to the total weight of the composition. The values are referring to the active content of the antioxidants.

### Examples

The following examples should illustrate the compositions of this invention, without, however, intending to limit the invention to these examples. The numerical values in the examples are percentages by weight based on the total weight of the compositions, and refer to their active content of each ingredients.

### Preparation method

The composition of the present invention may be prepared by any technique known or effective to prepare a hair styling composition. The process to prepare the composition of the present invention comprises conventional formulating and mixing techniques. The hair styling compositions, especially hair gel sprays, may be used as pump hair gel sprays or aerosol hair gel sprays.

Specifically, compositions of the present invention and a comparative composition are produced by the following procedure:
1. Preparation of phase A: Add Vinyl Caprolactam/VP/Dimethylaminoethyl Methacrylate Copolymer and sodium lauroyl sarcosinate into water and mix.
2. Preparation of phase B: Mix fragrance and solvent, preferably PEG-40 hydrogenated castor oil.
3. Add phase B to phase A.
4. Preparation of phase C: Dissolve the preservative(s) in ethanol, preferably a paraben and phenoxyethanol, more preferably methylparaben and phenoxyethanol.
5. Add phase C to combined phase A + B (step 3) and stir till a homogeneous composition is achieved.

Sodium lauroyl sarcosinate may be added at every stage of the preparation process, if necessary.

It is surprisingly found in the embodiments of the present invention that addition of sodium lauroyl sarcosinate, even in a small amount, may influence and improve the cloud point temperature of compositions containing Vinyl Caprolactam/VP/Dimethylaminoethyl Methacrylate Copolymer and preservatives.

Without being bound to any theory in the prior art, a possible mechanism how sodium lauroyl sarcosinate might work is that it, as a surfactant, increases solubilization of Vinyl Caprolactam/VP/Dimethylaminoethyl Methacrylate Copolymer and preservatives. Anion lauryl (12 carbon chain) would be best.

Main ingredients of the compositions of the present invention are listed in Table 1 as follows:

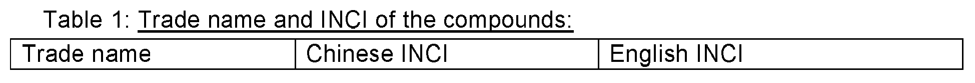

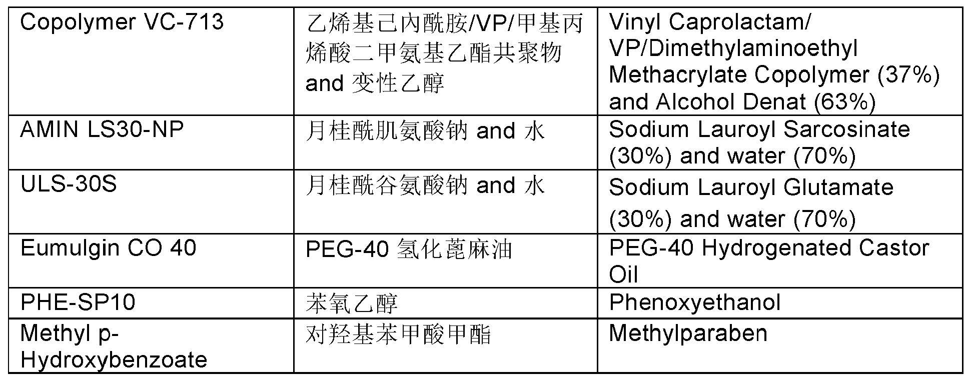

### Test method

A first series of experiments were conducted in order to analyze the influence of preservatives on the cloud point temperature of compositions containing a Vinyl Caprolactam/VP/Dimethylaminoethyl Methacrylate Copolymer (Table 2). Different preservatives, in combination or alone, in different amounts were analyzed. Additionally, the amount of the Vinyl Caprolactam/VP/Dimethylaminoethyl Methacrylate Copolymer was varied.

**Table 2**

| **Ingredients/Examples** | 1 | 2 | 3 | 4 | 5 | 6 | 7 | 8 | 9 | 10 |
|---|---|---|---|---|---|---|---|---|---|---|
| **Ethanol (95 %)** | 5 | 5 | 5 | 5 | 5 | 5 | 5 | 5 | 5 | 5 |
| **Vinyl Caprolactam/ VP/ Dimethylaminoethyl Methacrylate Copolymer (37 %)** | 4 | 4 | 4 | 4 | 4 | 5 | 5 | 5 | 5 | 5 |
| **Methylparaben** | 0.05 | 0.05 | | 0.1 | 0.1 | | 0.15 | 0.15 | | |
| **Phenoxyethanol** | 0.3 | | 0.3 | 0.5 | | 0.5 | 0.8 | | 0.8 | |
| **Water** | Ad 100 | Ad 100 | Ad 100 | Ad 100 | Ad 100 | Ad 100 | Ad 100 | Ad 100 | Ad 100 | Ad 100 |
| | | | | | | | | | | |
| **Turbidity (25°C)** | 7.4 | 9.5 | 6.4 | 2336 | 1268 | 5.0 | > 5000 | 2324 | 10.3 | 7.7 |
| **Turbidity (30°C)** | 7.3 | 8.6 | 6.3 | > 5000 | 2195 | 5.5 | > 5000 | 4255 | 41.8 | 7.3 |
| **Turbidity (35°C)** | 452 | 19.1 | 6.5 | > 5000 | > 5000 | 14 | > 5000 | > 5000 | 3415 | 6.5 |
| **Turbidity (40°C)** | 2398 | 1311 | 24 | > 5000 | > 5000 | 3064 | > 5000 | > 5000 | > 5000 | 6.2 |

| | | | | | | | | | | |
|---|---|---|---|---|---|---|---|---|---|---|
| > 5000: out of range. | | | | | | | | | | |

The amounts of ingredients are each referring to % by weight relative to the total weight of the compositions.

The turbidity values that are disclosed in the present specification have been determined using a turbidity measuring device (2100N TURBIDMETER from HACH), with distilled water with a value of NTU<0.1 serving as standard. The sample were undiluted. NTU is an abbreviation of Nephelometric Turbidity Unit.

Small turbidity values indicate clear compositions (values up to 30 NTU). High turbidity values are determined for compositions, which are cloudy and/or hazy (values greater than 100 NTU). The turbidity values of opaque compositions are not determinable. The turbidity of the compositions relating to examples 1 to 10 was determined at 30°C. It becomes apparent that a composition containing only the Vinyl Caprolactam/VP/Dimethylaminoethyl Methacrylate Copolymer and ethanol is clear at all temperatures analyzed (example 10). The addition of small amounts of preservatives influences the cloud point temperature as indicated by the turbidity values. Looking at the compositions according to example 4 and 5, the addition of methyl paraben alone (0.1 % by weight) or methyl paraben and Phenoxyethanol (0.1 % by weight and 0.5 % by weight, respectively) decreases the cloud point temperature resulting in hazy to opaque compositions, which are not according to the invention. The same holds true for compositions according to example 7 and 8. The addition of methyl paraben alone (0.15 % by weight) or methyl paraben and Phenoxyethanol (0.15 % by weight and 0.8 % by weight, respectively) decreases the cloud point temperature. The examples 4/5 and 7/8 differ in the amount of Vinyl Caprolactam/VP/Dimethylaminoethyl Methacrylate Copolymer, which is contained in the composition.

A second series of experiments was conducted analyzing the influence of the addition of sodium lauroyl sarcosinate on the cloud point temperature of the respective compositions (Table 3). The amounts of ethanol, methyl paraben, phenoxyethanol, fragrance and PEG-40 hydrogenated castor oil are fixed and resemble those suitable for compositions, which are intended for the market. The concentrations of the Vinyl Caprolactam/VP/Dimethylaminoethyl Methacrylate Copolymer and sodium lauroyl sarcosinate are varied.

**Table 3**

| **Ingredients/Examples** | **11** | **12** | **13** | **14** | **15** | **16** | **17** | **18** | **19** | **20** | **21** | **22** |
|---|---|---|---|---|---|---|---|---|---|---|---|---|
| **Ethanol (95%)** | 5 | 5 | 5 | 5 | 5 | 5 | 5 | 5 | 5 | 5 | 5 | 5 |
| **Methyl paraben** | 0.1 | 0.1 | 0.1 | 0.1 | 0.1 | 0.1 | 0.1 | 0.1 | 0.1 | 0.1 | 0.1 | 0.1 |
| **Phenoxyethanol** | 0.5 | 0.5 | 0.5 | 0.5 | 0.5 | 0.5 | 0.5 | 0.5 | 0.5 | 0.5 | 0.5 | 0.5 |
| **fragrance** | 0.1 | 0.1 | 0.1 | 0.1 | 0.1 | 0.1 | 0.1 | 0.1 | 0.1 | 0.1 | 0.1 | 0.1 |
| **PEG-40 Hydrogenated Castor Oil** | 0.4 | 0.4 | 0.4 | 0.4 | 0.4 | 0.4 | 0.4 | 0.4 | 0.4 | 0.4 | 0.4 | 0.4 |
| **Vinyl Caprolactam/ VP/Dimethylaminoethyl Methacrylate Copolymer (37%)** | 4 | 4 | 4 | 4 | 6 | 6 | 6 | 6 | 8 | 8 | 8 | 8 |
| ***Sodium Lauroyl sarcosinate*** | | ***0.6*** | ***1.0*** | ***1.4*** | | ***0.6*** | ***1.0*** | ***1.4*** | | ***0.6*** | ***1.0*** | ***1.4*** |
| **Water** | Ad 100 | Ad 100 | Ad 100 | Ad 100 | Ad 100 | Ad 100 | Ad 100 | Ad 100 | Ad 100 | Ad 100 | Ad 100 | Ad 100 |
| | | | | | | | | | | | | |
| **Turbidity (25°C)** | 1977 | 3.87 | 2.27 | 1.62 | 1309 | 3.84 | 2.38 | 1.85 | 778 | 3.55 | 4.22 | 2.11 |
| **Turbidity (30°C)** | 2623 | 4.03 | 2.33 | 1.67 | 3497 | 4.43 | 2.67 | 0.66 | 2946 | 5.31 | 2.77 | 2.26 |
| **Turbidity (35°C)** | >5000 | 563 | 2.66 | 1.98 | >5000 | 903 | 3.08 | 2.11 | >5000 | 962 | 3.67 | 2.55 |
| **Turbidity (40°C)** | >5000 | 1085 | 14.5 | 1.61 | >5000 | 2959 | 424 | 2.31 | >5000 | >5000 | 1024 | 3.69 |

| | | | | | | | | | | | | |
|---|---|---|---|---|---|---|---|---|---|---|---|---|
| > 5000: out of range. | | | | | | | | | | | | |

The amounts of ingredients are each referring to % by weight relative to the total weight of the compositions.

It becomes apparent that the addition of sodium lauroyl sarcosinate influences and improves the cloud point temperature of compositions containing Vinyl Caprolactam/VP/Dimethylaminoethyl Methacrylate Copolymer and preservatives. Even small amounts of sodium lauroyl sarcosinate (0.6 % by weight) elevate the cloud point temperature as can be seen from the turbidity values (examples 12, 16, 20).

## Claims

1. A cosmetic composition for styling hair, comprising:
1. a vinyl Caprolactam/VP/Dimethylaminoethyl Methacrylate Copolymer,
2. at least one salt of an acyl amino acid, salt of creatine and/or glutamate, and
3. at least one preservative, selected from the group of parabens and phenoxyethanol,
4. optionally at least one solvent.

2. The composition according to claim 1, **characterized in that** the Vinyl Caprolactam/VP/Dimethylaminoethyl Methacrylate Copolymer is contained in an amount of 0.1 to 8.0 % by weight, relative to the total weight of the composition.

3. The composition according to claim 2, **characterized in that** the Vinyl Caprolactam/VP/Dimethylaminoethyl Methacrylate Copolymer is contained in an amount of 0.3 to 4.0 % by weight, relative to the total weight of the composition.

4. The composition according to any of the proceeding claims, **characterized in that** the at least one salt of an acyl amino acid, salt of creatine and/or glutamate is contained in an amount of 0.1 to 3.0 % by weight, relative to the total weight of the composition.

5. The composition according to claim 4, **characterized in that** the at least one salt of an acyl amino acid, salt of creatine and/or glutamate is contained in an amount of 0.4 to 2.0 % by weight, relative to the total weight of the composition.

6. The composition according to any of the proceeding claims, **characterized in that** the at least one salt of an acyl amino acid is selected from the group of lauroyl glutamate, cocoyl glutamate, and lauroyl sarcosinate.

7. The composition according to any of the preceding claims, **characterized in that** the at least one salt of an acyl amino acid, salt of creatine and/or glutamate is a sodium or potassium salt, respectively.

8. The composition according to any of the proceeding claims, **characterized in that** at least one paraben and Phenoxyethanol are contained as preservatives.

9. The composition according to any of the proceeding claims, **characterized in that** the at least one paraben is contained in an amount of 0.01 to 0.5 % by weight, relative to the total weight of the composition.

10. The composition according to claim 9, **characterized in that** the at least one paraben is contained in an amount of 0.05 to 0.25 % by weight, relative to the total weight of the composition.

11. The composition according to any of the claims 8 to 10, **characterized in that** the at least one paraben is selected from methyl paraben and/or ethyl paraben.

12. The composition according to claim 8, **characterized in that** phenoxyethanol is contained in an amount of 0.1 to 1.5 % by weight, relative to the total weight of the composition.

13. The composition according to claim 12, **characterized in that** phenoxyethanol is contained in an amount of 0.2 to 1.0 % by weight, relative to the total weight of the composition.

14. The composition according to any of the proceeding claims, **characterized in that** the composition further comprises at least one antioxidant and the composition comprises a balance amount of water, optionally ethanol and/or PEG-40 hydrogenated castor oil, so that all the components amount to 100% by weight.

15. The composition according to any of the preceding claims, **characterized in that** the composition is a hair gel spray.

16. Use of at least one salt of an acyl amino acid, salt of creatine and/or glutamate to elevate the cloud point temperature of a composition containing a Vinyl Caprolactam/VP/Dimethylaminoethyl Methacrylate Copolymer and at least one preservative, selected from the group of parabens and phenoxyethanol.

17. Use according to claim 16, **characterized in that** the at least one salt of an acyl amino acid, salt of creatine and/or glutamate is a sodium or potassium salt, respectively.

18. Use of the composition according to any of claims 1-15 for styling and caring hair.

19. Use according to claim 18, **characterized in that** the styling of hair provides the hair with a long-lasting hold.

## Patentansprüche

1. Kosmetische Zusammensetzung zum Stylen der Haare, umfassend:
1. ein Vinylcaprolactam/VP/Dimethylaminoethylmethacrylat-Copolymer,
2. mindestens ein Salz einer Acylaminosäure, Salz von Kreatin und/oder Glutamat und
3. mindestens einen Konservierungsstoff, ausgewählt aus der Gruppe der Parabene und Phenoxyethanol,
4. gegebenenfalls mindestens ein Lösungsmittel.

2. Zusammensetzung nach Anspruch 1, **dadurch gekennzeichnet, dass** das Vinylcaprolactam/VP/Dimethylaminoethylmethacrylat-Copolymer in einer Menge von 0,1 bis 8,0 Gew.-%, bezogen auf das Gesamtgewicht der Zusammensetzung, enthalten ist.

3. Zusammensetzung nach Anspruch 2, **dadurch gekennzeichnet, dass** das Vinylcaprolactam/VP/Dimethylaminoethylmethacrylat-Copolymer in einer Menge von 0,3 bis 4,0 Gew.-%, bezogen auf das Gesamtgewicht der Zusammensetzung, enthalten ist.

4. Zusammensetzung nach einem der vorhergehenden Ansprüche, **dadurch gekennzeichnet, dass** das mindestens eine Salz einer Acylaminosäure, Salz von Kreatin und/oder Glutamat in einer Menge von 0,1 bis 3,0 Gew.-%, bezogen auf das Gesamtgewicht der Zusammensetzung, enthalten ist.

5. Zusammensetzung nach Anspruch 4, **dadurch gekennzeichnet, dass** das mindestens eine Salz einer Acylaminosäure, Salz von Kreatin und/oder Glutamat in einer Menge von 0,4 bis 2,0 Gew.-%, bezogen auf das Gesamtgewicht der Zusammensetzung, enthalten ist.

6. Zusammensetzung nach einem der vorhergehenden Ansprüche, **dadurch gekennzeichnet, dass** das mindestens eine Salz einer Acylaminosäure aus der Gruppe Laurylglutamat, Cocoylglutamat und Lauroylsarcosinat ausgewählt ist.

7. Zusammensetzung nach einem der vorhergehenden Ansprüche, **dadurch gekennzeichnet, dass** es sich bei dem mindestens einen Salz einer Acylaminosäure, Salz von Kreatin und/oder Glutamat um ein Natrium- bzw. Kaliumsalz handelt.

8. Zusammensetzung nach einem der vorhergehenden Ansprüche, **dadurch gekennzeichnet, dass** mindestens ein Paraben und Phenoxyethanol als Konservierungsstoffe enthalten sind.

9. Zusammensetzung nach einem der vorhergehenden Ansprüche, **dadurch gekennzeichnet, dass** das mindestens eine Paraben in einer Menge von 0,01 bis 0,5 Gew.-%, bezogen auf das Gesamtgewicht der Zusammensetzung, enthalten ist.

10. Zusammensetzung nach Anspruch 9, **dadurch gekennzeichnet, dass** das mindestens eine Paraben in einer Menge von 0,05 bis 0,25 Gew.-%, bezogen auf das Gesamtgewicht der Zusammensetzung, enthalten ist.

11. Zusammensetzung nach einem der Ansprüche 8 bis 10, **dadurch gekennzeichnet, dass** das mindestens eine Paraben aus Methylparaben und/oder Ethylparaben ausgewählt ist.

12. Zusammensetzung nach Anspruch 8, **dadurch gekennzeichnet, dass** das Phenoxyethanol in einer Menge von 0,1 bis 1,5 Gew.-%, bezogen auf das Gesamtgewicht der Zusammensetzung, enthalten ist.

13. Zusammensetzung nach Anspruch 12, **dadurch gekennzeichnet, dass** das Phenoxyethanol in einer Menge von 0,2 bis 1,0 Gew.-%, bezogen auf das Gesamtgewicht der Zusammensetzung, enthalten ist.

14. Zusammensetzung nach einem der vorhergehenden Ansprüche, **dadurch gekennzeichnet, dass** die Zusammensetzung ferner mindestens ein Antioxidans umfasst und die Zusammensetzung eine solche Restmenge an Wasser, gegebenenfalls Ethanol und/oder PEG-40-hydriertem Rizinusöl umfasst, dass die Summe aller Komponenten 100 Gew.-% beträgt.

15. Zusammensetzung nach einem der vorhergehenden Ansprüche, **dadurch gekennzeichnet, dass** es sich bei der Zusammensetzung um ein Haargelspray handelt.

16. Verwendung mindestens eines Salzes einer Acylaminosäure, Salzes von Kreatin und/oder Glutamats zur Erhöhung der Trübungspunkttemperatur einer Zusammensetzung, die ein Vinylcaprolactam/VP/Dimethylaminoethylmethacrylat-Copolymer und mindestens einen Konservierungsstoff, der aus der Gruppe der Parabene und Phenoxyethanol ausgewählt ist, enthält.

17. Verwendung nach Anspruch 16, **dadurch gekennzeichnet, dass** es sich bei dem mindestens einen Salz einer Acylaminosäure, Salz von Kreatin und/oder Glutamat um ein Natrium- bzw. Kaliumsalz handelt.

18. Verwendung der Zusammensetzung nach einem der Ansprüche 1-15 zum Stylen und Pflegen der Haare.

19. Verwendung nach Anspruch 18, **dadurch gekennzeichnet, dass** das Stylen der Haare den Haaren einen lang andauernden Halt verleiht.

## Revendications

1. Composition cosmétique pour le coiffage des cheveux, comprenant :
1) un copolymère de vinylcaprolactame/VP/méthacrylate de diméthylaminoéthyle,
2) au moins un sel d'un acide aminé acylé, sel de créatine et/ou glutamate, et
3) au moins un conservateur, choisi dans le groupe des parabènes et du phénoxyéthanol,
4) éventuellement au moins un solvant.

2. Composition selon la revendication 1, **caractérisée en ce que** le copolymère de vinylcaprolactame/VP/méthacrylate de diméthylaminoéthyle est contenu en une quantité de 0,1 à 8,0 % en poids, par rapport au poids total de la composition.

3. Composition selon la revendication 2, **caractérisée en ce que** le copolymère de vinylcaprolactame/VP/méthacrylate de diméthylaminoéthyle est contenu en une quantité de 0,3 à 4,0 % en poids, par rapport au poids total de la composition.

4. Composition selon l'une quelconque des revendications précédentes, **caractérisée en ce que** l'au moins un sel d'un acide aminé acylé, sel de créatine et/ou glutamate est contenu en une quantité de 0,1 à 3,0 % en poids, par rapport au poids total de la composition.

5. Composition selon la revendication 4, **caractérisée en ce que** l'au moins un sel d'un acide aminé acylé, sel de créatine et/ou glutamate est contenu en une quantité de 0,4 à 2,0 % en poids, par rapport au poids total de la composition.

6. Composition selon l'une quelconque des revendications précédentes, **caractérisée en ce que** l'au moins un sel d'un acide aminé acylé est choisi dans le groupe du glutamate de lauroyle, du glutamate de cocoyle et du sarcosinate de lauroyle.

7. Composition selon l'une quelconque des revendications précédentes, **caractérisée en ce que** l'au moins un sel d'un acide aminé acylé, sel de créatine et/ou glutamate est un sel de sodium ou de potassium, respectivement.

8. Composition selon l'une quelconque des revendications précédentes, **caractérisée en ce qu'**au moins un parabène et du phénoxyéthanol sont contenus en tant que conservateurs.

9. Composition selon l'une quelconque des revendications précédentes, **caractérisée en ce que** l'au moins un parabène est contenu en une quantité de 0,01 à 0,5 % en poids, par rapport au poids total de la composition.

10. Composition selon la revendication 9, **caractérisée en ce que** l'au moins un parabène est contenu en une quantité de 0,05 à 0,25 % en poids, par rapport au poids total de la composition.

11. Composition selon l'une quelconque des revendications 8 à 10, **caractérisée en ce que** l'au moins un parabène est choisi parmi le méthylparabène et/ou l'éthylparabène.

12. Composition selon la revendication 8, **caractérisée en ce que** le phénoxyéthanol est contenu en une quantité de 0,1 à 1,5 % en poids, par rapport au poids total de la composition.

13. Composition selon la revendication 12, **caractérisée en ce que** le phénoxyéthanol est contenu en une quantité de 0,2 à 1,0 % en poids, par rapport au poids total de la composition.

14. Composition selon l'une quelconque des revendications précédentes, **caractérisée en ce que** la composition comprend en outre au moins un antioxydant et la composition comprend une quantité restante d'eau, éventuellement d'éthanol et/ou d'huile de ricin hydrogénée PEG-40, de sorte que tous les composants totalisent 100 % en poids.

15. Composition selon l'une quelconque des revendications précédentes, **caractérisée en ce que** la composition est un spray de gel capillaire.

16. Utilisation d'au moins un sel d'un acide aminé acylé, sel de créatine et/ou glutamate pour élever la température de point de trouble d'une composition contenant un copolymère de vinylcaprolactame/VP/méthacrylate de diméthylaminoéthyle et au moins un conservateur, choisi dans le groupe des parabènes et du phénoxyéthanol.

17. Utilisation selon la revendication 16, **caractérisée en ce que** l'au moins un sel d'un acide aminé acylé, sel de créatine et/ou glutamate est un sel de sodium ou de potassium, respectivement.

18. Utilisation de la composition selon l'une quelconque des revendications 1 à 15 pour le coiffage et l'entretien de cheveux.

19. Utilisation selon la revendication 18, **caractérisée en ce que** le coiffage des cheveux procure aux cheveux une tenue de longue durée.
